# EUROPEAN PATENT SPECIFICATION

(11) **EP 4 608 239 B1**
(45) Date of publication and mention of the grant of the patent: **29.07.2026**
(21) Application number: 23783369.4
(22) Date of filing: 29.09.2023
(51) Int. Cl.: A61B 5/00, A61B 5/145, A61B 5/1486

(54) **ANALYTE MONITORING SYSTEM**
ANALYTÜBERWACHUNGSSYSTEM
SYSTÈME DE SURVEILLANCE D'ANALYTE

(30) Priority: 26.10.2022 GB 202215851
(43) Date of publication of application: 03.09.2025
(73) Proprietor: Glucorx Technologies Limited, Dingwall IV15 9QF (GB)
(72) Inventor: MURRAY-SCOTT, Cerys Rohann, Dingwall IV15 9QF (GB); PITTS, Dave, Dingwall IV15 9QF (GB); MOFFAT, Jim, Dingwall IV15 9QF (GB); MCCULLOCH, Andrew, Suffolk IP13 9EZ (GB)
(74) Representative: Marks & Clerk LLP
(86) International application number: PCT/EP2023/077172
(87) International publication number: WO 2024/088700

(56) References cited:
- EP-A1- 3 960 072
- EP-B1- 2 393 417
- WO-A1-2020/027422

## Description

### Field of Invention

The present disclosure relates an analyte monitoring system, a sensor module for use with an analyte monitoring system, and a method of activating a sensor module of an analyte monitoring system.

### Background

Traditionally, monitoring blood glucose levels of a patient involved a process where a finger prick blood test obtained a small drop of blood that was placed on a test strip that inserted into glucometer. The glucometer read the strip and provided a digital reading of the individual's blood sugar level.

Recently, finger prick blood tests have been replaced by insertable (implantable), in vivo, analyte sensors that are inserted into the skin of the patient where they remain at all times, enabling substantially continuous measurements to be taken, which is advantageous compared to finger prick tests that provide only snapshot readings at a small number of times a day. These types of implantable analyte sensors are typically coupled to a sensor module having a housing placed on the surface of the patient's skin. For example, the rearwardly protruding part of the analyte sensor is inserted into the skin-facing surface of the housing the sensor module, where it is coupled inside the sensor module to control electronics. This ensures only the sensor module itself is visibly exposed to the outside environment and the entry point of the insertable analyte sensor in the skin is at least partially protected underneath the sensor module. The sensor module control electronics process measurement signals from the inserted sensor and transmit any relevant information to, for example, the patient's smartphone or other mobile device. These types of systems are sometimes known as continuous analyte monitoring systems.

Typically, continuous analyte monitoring systems require the analyte sensor to be replaced at predetermined intervals and this may require the patient to insert the analyte sensor themselves in an unsupervised environment. In order to simplify this process, known analyte monitoring systems are provided with an inserter device which applies a predetermined amount of force to the insertable analyte sensor to safely insert it into the patient's skin, and at the same time to position any accompanying sensor module at the surface of the patient's skin. EP2393417B1 proposes a continuous analyte monitoring system.

In known continuous analyte monitoring systems, such as that of EP2393417B1, the sensor module is initially inside of the inserter device. An adhesive pad or patch (sometimes referred to as an epidermal support patch) is provided on the skin-facing end of the inserter device. During use, the inserter device inserts the analyte sensor into the skin, places the sensor module onto the adhesive pad which is thereby secured to the patient's skin.

WO2020/027422 A1 generally describes a continuous blood glucose measurement body attachment unit which is manufactured in an assembled state in an applicator so as to minimize separate additional operations.

The circuitry in the sensor module of known continuous analyte monitoring systems is powered by a battery. When such systems are in storage prior to use, a plastic tab or pin stops the battery from touching its contacts on the sensor module circuitry to prevent system from switching on. Once the sensor module is secured to the patient's skin, the plastic tab or pin may be pulled out, allowing the battery to touch its contact pads and thereby activating the sensor module. This is inconvenient as it requires the user to take an additional action, namely manually pulling on the plastic tab, to switch on the sensor module and the user may forget to take this action. After removal of the plastic tab or pin, an opening in the sensor module is typically left exposed, through which moisture or other contaminant may enter.

An improved analyte monitoring system, sensor module and corresponding method of activating a sensor module is desired.

### Summary

In general terms, the present disclosure is directed to a sensor module with an opening, for example on the skin-facing surface, covered by an elastically deformable cover through which the controlling circuitry of the sensor module can be activated. Advantageously, unlike in known systems where a tab or pin is pulled out to activate the device leaving an exposed opening, the elastically deformable allows the circuitry to be activated without leaving any exposed openings.

Thus, according to a first aspect of the disclosure, there is provided an analyte monitoring system comprising: a skin-implantable analyte sensor; and a sensor module coupled to the skin-implantable analyte sensor, wherein the sensor module comprises: a housing having an opening in a surface thereof; a cover for covering the opening, the cover comprising an elastically deformable material; and circuitry disposed in the housing, and wherein, responsive to application of a force on a surface of the cover, the cover is configured to elastically deform into the housing to activate the circuitry.

Optionally, the opening in the housing is in a surface of the housing that faces the skin of a user when in use.

Advantageously, positioning the opening and cover through which the circuitry is activated on the skin-facing surface of a user (as opposed to on the side or outward facing surfaces of the housing) ensures only the stronger, non-deformable outer surface of the housing is exposed to the environment or accidental touching by the user. This is because the sensor module is secured to the user's skin with adhesive, for example an adhesive patch, which may completely cover and protect the opening and elastically deformable cover when in use.

Optionally, wherein after said application of said force ends, the elastically deformed cover is configured to return to its original shape, substantially flush with said surface of the housing.

Advantageously, unlike in systems where an opening is left exposed, when the elastically deformable cover returns to its original shape i.e. substantially flush with the skin-facing surface of the sensor module, it provides a substantially continuous surface with which to adhere the sensor module with adhesive (for example an adhesive patch) to the surface of the skin of the user. As a result, the surface area with adhesive is greater than it would be for a non-continuous surface and the adhesive bond is thus stronger. Further, the elastically deformable cover not only enables the opening to be provided on the skin-facing surface in a way that protects the opening from the environment and provides a stronger bond surface, but it also synergistically enables the cap of an inserter device (as is described later below) to be used to elastically deform the cover to activate the circuitry without the need for any other switch-on mechanism in the inserter device. As a result, the analyte monitoring system is less complex internally and accordingly cheaper to manufacture compared to known systems.

Optionally, the circuitry comprises a momentary switch activated by the elastic deformation of the elastically deformable cover into the housing, and wherein the circuitry is configured to perform a start-up routine responsive to activation of the momentary switch.

Advantageously, providing the switch-on mechanism that activates the start-up routine of the sensor module as a momentary switch means it is not necessary for the force, pressure, or movement that activates the circuitry to be continuous so there is no need for a spring, cantilever or other such mechanism to be used thereby simplifying the sensor module and allowing it to be manufactured more cheaply compared to known systems.

Optionally, the analyte sensor comprises a deformable portion configured to be deformed into contact with the circuitry responsive to a force applied thereto by the elastically deformable cover, thereby activating the momentary switch.

Advantageously, using the analyte sensor to provide a deformable portion onto which the elastically deformable cover presses instead of, for example, a deformable portion that is part of the housing of the sensor module substantially simplifies the housing. In particular, implementing deformable portions in a complex three-dimensional shape such as an injection-moulded housing of a sensor module is challenging and an expensive undertaking. In contrast, implementing a deformable portion on the relatively simpler shape of the analyte sensor is easier and cheaper. Thus, the inventors have realised that moving functionality of the switch mechanism (i.e. a deformable portion) into the analyte sensor instead of having it as part of the housing results in a simpler and more cheaply and easily manufactured sensor module.

Optionally, the deformable portion of the analyte sensor comprises a contact pad, wherein the momentary switch comprises a contact pad, and wherein said momentary switch is configured to be activated by contact between the contact pad of the deformable portion of the analyte sensor and the contact pad of the momentary switch.

Advantageously, activating the momentary switch by temporary contact between the electrically conductive contact pad of the analyte sensor and a corresponding pad on the momentary switch instead of using other switch temporarily completes a switch-on circuit in the circuitry. Synergistically, not only does moving some functionality of the switch mechanism onto the analyte sensor module simplify the housing due to the absence of a deformable switch portion as described above, but moving the conductive pad functionality from the housing onto the analyte sensor itself avoids the need for a conductive material deposition step during manufacture of the housing. In contrast, analyte sensors already have a conductive material deposition step during manufacture so including a deposition step during manufacture of the analyte sensor to add a switch-on contact pad provides a simpler solution. By way of non-limiting example, the switch-on circuit may run from a battery of the circuitry to the momentary switch contact pad, through the analyte sensor contact pad, and back to the circuitry by one or more other contact pads of the analyte sensor which are also in contact with the circuitry. Other suitable switch-on circuits that are activated by momentary switches are also envisaged as will be appreciated by the skilled person. The circuitry may for example comprise one or more processing resources, for example ASICs to detect a switch-on signal from the momentary switch and thereby start a start-up routine of the circuitry using suitable computer programme instructions stored on a memory of the circuitry. As will be appreciated, the components of the circuitry and the connections between them may be provided on a printed circuit board.

Optionally, an inside surface of the housing comprises a recess partially contiguous with an edge of the opening in the housing, the recess configured to receive a portion of the analyte sensor therein.

Advantageously, positioning the recess for receiving the analyte sensor immediately adjacent or contiguous with the opening enables the deformable portion of the analyte sensor to be used to activate the temporary switch as the elastic deformation of the cover which applies a force to the deformable portion of the analyte sensor occurs through said opening.

Optionally, the deformable portion of the analyte sensor when positioned in the recess is disposed over the opening in the housing.

Optionally, the analyte sensor may comprise an aperture, opening or eyelet configured to receive a protrusion of the housing, to thereby positionally secure the analyte sensor within the housing. A shape of the eyelet may be e.g. a circle or ellipse, or may be a shape comprising one or more corners or otherwise pointed ends (such as an oval or triangle).

Advantageously, as described above, positioning the deformable portion of the analyte sensor over the opening ensures the elastic deformation of the cover applies a force to the deformable portion of the analyte sensor, thereby causing it to contact the momentary switch and thus commence the circuitry start up routine.

Optionally, a surface of the recess or an inside surface of the cover which itself may cover part of the recess comprises one or more raised portions configured to provide a reaction force against the analyte sensor when the analyte sensor is pressed into the recess or onto the inside surface of the cover.

As will be appreciated, in addition to the switch-on contact pad of the analyte sensor that is described above, the analyte sensor may have additional contact pads that are coupled to corresponding contact pads on the circuitry, for example on a skin-facing surface of the PCB. These additional contact pads provide the electrical connection between the sensing components of the analyte sensor implanted into the user's skin, and the circuitry. Thus, unlike the switch-on contact pad that only requires a temporary contact with the circuitry, the additional contact pads require continuous and uninterrupted connection to the circuitry, and thus contact with the circuitry contact pads, during use. In order to ensure this contact is reliable, the housing and/or an inside surface of the elastomeric cover covering the recess is provided with raised portions at positions corresponding to the positions of the contact pads on the analyte sensor. When the analyte sensor is placed and pressed into the recess in the sensor module and the housing is closed, the compression force of the housing parts being joined together is directed via the raised portions onto a skin-facing surface of the analyte sensor at the positions of the contact pads, thereby forcing the contact pads on the opposite surface directly into contact with contact pads on the circuitry. The raised portions accordingly function as force or energy directors by ensuring the compression force of closing the housing is correctly directed to the location of the contact pads.

Optionally, the continuous analyte monitoring system comprises a compressible layer of material positioned over at least part of the recess and configured to contribute to the reaction force and to distribute the reaction force across a surface of the analyte sensor.

Advantageously, the compressive layer increases the force with which the contact pads of the analyte sensor are held in reliable contact with the corresponding contact pads on the sensor module as its compression will exert a reaction force against the analyte sensor. Further, it will distribute this force more evenly over the analyte sensor to avoid any damage to the analyte sensor surface. The compressible layer may comprise a spongy and/or elastomeric material.

Optionally, the housing has a further opening configured for receiving a portion of the analyte sensor therethrough, and wherein the further opening is sealed with an O-ring seal.

Advantageously, the further opening (through which the pointed portion of the analyte sensor protrudes from the housing and into the skin of the user) is made waterproof by providing an O-ring seal, for example made of an elastomeric material. Thus the opening for switching on the sensor module is waterproof by way of the elastically deformable cover, and the opening for the analyte sensor pointed portion is waterproof by way of an O-ring.

Optionally, the compressible layer and/or, where present, the O-ring seal, and/or where present the one or more raised portions are integral with and comprise the same material as the cover.

Advantageously, providing the compressible layer, the O-ring seal, and/or the one or more raised portions as a single, in the same material as, and integral with, the housing is substantially simplified thereby making it cheaper and easier to manufacture with, for example, injection moulding. Instead, these features are formed in an elastomeric material in a single step, something which is easier to do with elastomeric material than the hard plastic of the housing.

Optionally, the opening defines a partial ring-like shape across a portion of the surface of the housing.

Advantageously, a partial ring-like shaped opening i.e. an opening that follows a generally circular path facilitates the use of a circular or rotating motion to be used to deform the elastic cover and activate the circuitry. For example, as will be described below, the rotating motion of the removal of a cap of an inserter device may be used to switch on the sensor module.

Optionally, the analyte monitoring system comprises: an inserter device configured to implant the analyte sensor into the skin of a user and position the sensor module onto a surface of the skin of the user, the inserter device comprising a body and a removable cap, wherein a space enclosed by the body and the cap is configured to receive the sensor module and analyte sensor therein before use, wherein the cap comprises an arm configured to apply a force to a surface of the cover of the sensor during removal of the cap from the body to elastically deform the cover into the housing to activate the circuitry.

Advantageously, incorporating an arm configured to deform the elastically deformable cover into a cap which performs said deforming during removal of the cap means the user cannot forget to switch on the sensor module as they would be unable to access the sensor module until the cap is removed. The number of instances of user error are accordingly reduced.

Optionally, the removable cap is configured to rotate relative to the body of the inserter device during removal to cause the arm to apply said force to the surface of the cover.

Advantageously, as described above, a circular opening in the housing is synergistically compatible with the rotating motion of a twist-off cap, which are easy for a user to use. Thus, the switch-on of the sensor module is made easy for the user because they need not learn any unusual motion or skill to switch on the device but only have to remove the cap.

Optionally, the body of the inserter device comprises a thread on an inside surface thereof configured for engagement with a lug on the removable cap, and wherein during removal of the removable cap by rotation, the lug is configured to follow the thread thereby moving the removable cap and arm in a direction towards the sensor module and applying a force with the arm to said surface of the cover to activate the circuitry.

Advantageously, the lug and thread mechanism allow the rotational movement applied by the user to the cap to be partially converted into a linear force in the arm of the cap in the direction along the axis of rotation (i.e. arm is moved in the direction of the sensor module and into the opening of the housing, thereby elastically deforming the cover and activating the sensor module).

Optionally, the inserter device further comprises a carriage for receiving the sensor module therein before use, and a carriage release sleeve, the carriage release sleeve being configured to release the carriage under a force to move the sensor module onto the surface of the skin of the user.

Advantageously, it is envisaged that the inserter device is activated by pressing the carriage release sleeve onto the patient's skin rather than requiring a button or other activation mechanism, thereby simplifying the internal structure of the inserter device. For example, the carriage release sleeve may protrude partially from the skin-facing end of the body. Further, it is envisaged that the inserter device may be a spring-less inserter device, i.e. a device which relies solely on the force of the user to implant the analyte sensor instead of using a spring to apply the force. Thus, the user removes the cap to activate the sensor module circuitry, aligns the inserter device on the skin by placing the carriage release sleeve on the skin (or on an adhesive patch if provided), and pushes down with a force onto a rear surface of the inserter device body. This causes forward movement of the body (with carriage and sensor module therein) relative to the carriage release sleeve placed on the skin until the sensor module reaches the skin-facing end of the inserter device where pointed end of the analyte sensor is implanted into the skin, and the sensor module is secured to the skin for example with an adhesive patch.

Optionally, the analyte sensor is a blood glucose sensor.

Whilst it is envisaged that the analyte sensor of the present disclosure is a blood glucose sensor. It is also envisaged that the sensor may be used to measure other analytes.

Optionally, the sensor module is secured to the user's skin with an adhesive patch.

Advantageously, use of an adhesive patch ensures the sensor module and implanted analyte sensor remain secured to the user following use of the inserter device.

It is further envisaged that the above advantages are applicable when the sensor module is taken as a standalone device. Thus, according to a second aspect of the present disclosure, there is provided a sensor module for use with an analyte monitoring system with a skin-implantable analyte sensor, the sensor module comprising: a housing having an opening in a surface thereof; a cover for covering the opening, the cover comprising an elastically deformable material; and circuitry disposed in the housing, and wherein, responsive to application of a force on a surface of the cover, the cover is configured to elastically deform into the housing to activate the circuitry.

Optionally, the opening in the housing is in a surface of the housing that faces the skin of a user when in use.

Optionally, after said application of said force ends, the elastically deformed cover is configured to return to its original shape, substantially flush with said surface of the housing.

It is further envisaged that the above advantages are applicable to a corresponding method of using the analyte monitoring system. Thus, according to a third aspect, there is provided a method of activating a sensor module of an analyte monitoring system with a skin-implantable analyte sensor and inserter device, wherein the sensor module comprises: a housing having an opening in a surface thereof, a cover for covering the opening, the cover comprising an elastically deformable material; and circuitry disposed in the housing, and wherein the method comprises applying a force to a surface of the cover to elastically deform the cover into the housing to activate the circuitry.

Optionally, the analyte monitoring system comprises an inserter device configured to implant the analyte sensor into the skin of a user and position the sensor module onto a surface of the skin of the user, wherein the inserter device comprises a body and a removable cap enclosing a space for receive the sensor module and analyte sensor therein before use, the method comprising: applying said force to the surface of the cover during removal of the removable cap from the body by rotating the removable cap relative to the body to force an arm of removable cap against the cover.

### Brief Description of the Drawings

These and other aspects will now be described, by way of example only, with reference to the accompanying figures in which:
Figure 1a illustratively shows an exploded view of an analyte monitoring system according to the present disclosure.
Figure 1b illustratively shows the exploded view of Figure 1a from a different angle.
Figure 2a illustratively shows a view of a segment of a housing of a sensor module of the present disclosure.
Figure 2b illustratively shows a view of a segment of a housing of a sensor module of the present disclosure.
Figure 3 illustratively shows a view of a segment of a housing of a sensor module of the present disclosure.
Figure 4 illustratively shows a view of a segment of a housing of a sensor module of the present disclosure.
Figure 5a illustratively shows an exploded view of an analyte monitoring system according to the present disclosure.
Figure 5b illustratively shows a section view of an assembled analyte monitoring system according to the present disclosure.
Figure 6a illustratively shows the operation of a switch-on mechanism according to the present disclosure.
Figure 6b illustratively shows the operation of a switch-on mechanism according to the present disclosure.
Figure 6c illustratively shows the operation of a switch-on mechanism according to the present disclosure.
Figure 6d illustratively shows the operation of a switch-on mechanism according to the present disclosure.
Figure 7a illustratively shows an exploded view of an analyte monitoring system according to the present disclosure.
Figure 7b illustratively shows the exploded view of Figure 7a from a different angle.
Figure 8a illustratively shows an analyte sensor according to the present disclosure.
Figure 8b illustratively shows the analyte sensor of Figure 8a from a different angle.
Figures 9a-d illustratively show the insertion of an analyte sensor into an analyte monitoring system according to the present disclosure.
Figures 10a and b illustratively show the insertion of system circuitry into an analyte monitoring system according to the present disclosure.
Figure 11 illustratively shows example circuitry for an analyte monitoring system according to the present disclosure.
Figures 12a and b illustratively show the insertion of system circuitry into an analyte monitoring system according to the present disclosure.

### Detailed Description of the Drawings

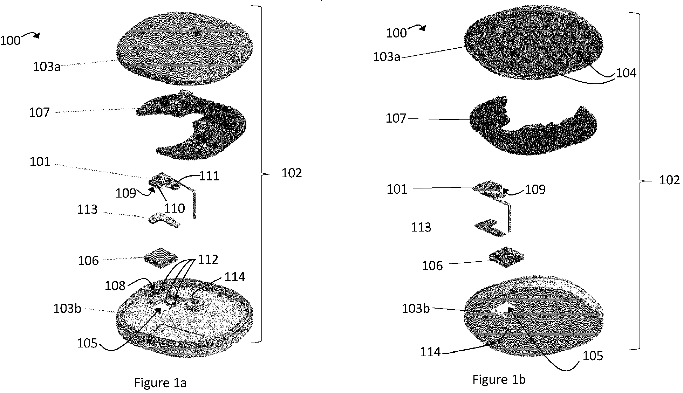

Figure 1a illustratively shows an exploded view of an analyte monitoring system 100 according to the present disclosure. The analyte monitoring system comprises a skin implantable analyte sensor 101, and a sensor module 102 coupled to the skin-implantable analyte sensor 101. The sensor module 102 comprises a housing 103 that comprises two segments 103a, 103b, for example injection moulded, plastic segments joined and sealed together during assembly using a push-fit connection or adhesive. One of the segments 103b has an internal surface provided with support structures to enhance structural integrity of the housing 103. The housing 103 is approximately disc or rounded square shaped and is configured to be placed on a surface of the user's skin, or on an adhesive patch that is on the skin, during use. An outer surface of one of the segments 103b of the housing 103, is provided with an opening 105 therethrough.

The opening 105 is covered by a cover 106 comprising an elastically deformable material that is configured to deform when a force is applied to a surface thereof. The elastically deformable material may be, for example, an elastomer. The opening 105 and matching cover 106 shown in Figures 1 and 1b are square shaped. However, it is envisaged that other shapes may be used, including a partial ring-like shape that extends along an approximately circular path through around the surface of the housing segment 103b.

The sensor module 102 is further provided with circuitry 107, for example a printed circuit board (PCB) having electronic components thereon including, for example, a battery, processing resources such as ASICs, a memory, a communication's interface for transmitting readings to a user interface, and other circuitry components that are known to skilled person.

The segment of the housing 103b is also provided with a recess 108 or slot in an inner surface thereof for receiving the analyte sensor 101. The recess is accordingly shaped to match the shape of the analyte sensor 101 to provide a snug fit for a portion of the analyte sensor 101, and leaving another portion of the analyte sensor 101 exposed through the opening 105. This allows a deformable portion 109 of the analyte sensor 101, and specifically a contact pad 110 on an upper surface of the deformable portion 109, to be temporarily forced into contact with a corresponding contact pad (not shown) on an underside of the PCB responsive to a force applied through the elastically deformable cover. The temporary contact between the contact pads 110 commences a start-up routine of the circuitry 107.

The analyte sensor 101 is further provided with additional contact pads 111, for example one, two, three or more contact pads, which are configured to be continuously connected to corresponding contact pads (not shown) on the underside of the PCB. These additional contact pads 111 are intended to couple the sensing portion of the analyte sensor 101 with the circuitry 107.

As shown in Figures 1a and 1b, the analyte sensor 101 has a pointed portion configured to protrude from the housing and be implanted into the skin do the user, and a flat portion, substantially perpendicular to the pointed portion, comprising the contact pads 110, 111 and deformable portion.

A surface of the recess 108 is further provided with raised portions 112, for example formed during an injection moulding process, located substantially under the contact pads 111 which are intended to continuously be in connection with corresponding contact pads on the PCB. When the analyte sensor 101 is positioned in the recess 108 and the housing segments 103a, 103b joined together during assembly, the raised portions 112 provide a reaction force against the analyte sensor 101 thereby compressing the contact pads 111 against the corresponding contact pads to maintain a reliable link between them. To prevent damage to the analyte sensor 101 from the raised portions 112, a compressible layer of material 113, shaped approximately like the analyte sensor 101, may be provided between the raised portions 112 and the analyte sensor 101 to distribute the force from the raised portions 112 more evenly across the surface of the analyte sensor 101. The compressible layer of material 113 may be an elastomer or sponge material, which may contribute to the reaction force upon compression.

The segment of the housing 103b may further be provided with an additional opening 114 through which the pointed portion of the analyte sensor may protrude from the housing 103.

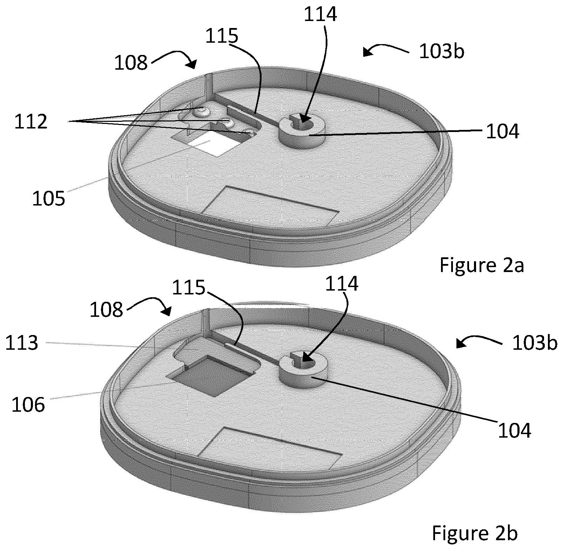

Figure 2a illustratively shows a close up view of a segment 103b of the housing 103 of the sensor module of Figures 1a and 1b. As is shown in Figure 2a, the recess 108 is contiguous with the opening 105, for example it is immediately adjacent the opening 105. The recess 108 matches the shape of the analyte sensor 101 so accordingly includes a channel 115 to fit the joining portion of the analyte sensor 101 between the pointed and flat portions. Figure 2a also shows a supporting structure 104 which partially surrounds the additional opening 114 of the housing segment 103b to minimise the area through which moisture might otherwise enter the additional opening 114. An O-ring seal (not shown) may surround the supporting structure 104.

Figure 2b corresponds to Figure 2a but now also showing the intended positioning of the elastically deformable cover 106 and compressible layer 113 inside the recess 108 when assembled.

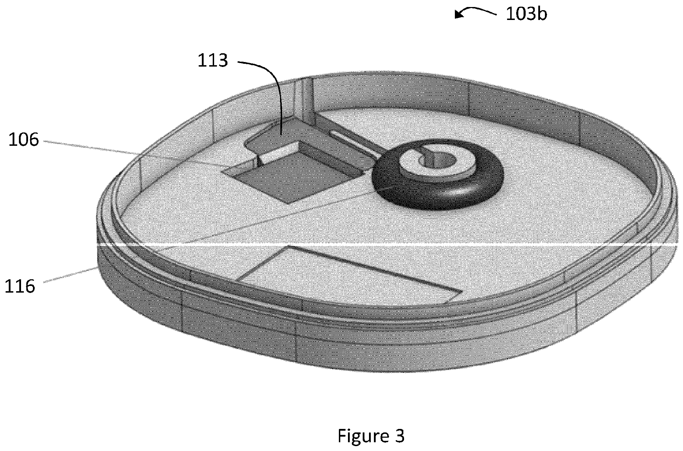

Figure 3 illustratively shows a view of a segment 103b of the housing 103 that corresponds to that shown in Figures 1a-2b except in this case, the compressible layer of material is integral with the cover 106 and thus made of the same material. In other words, the cover 106 may cover not only the opening 105 but also the raised portions 112 in the configuration shown in Figure 3. The O-ring seal 116 is also visible in Figure 3.

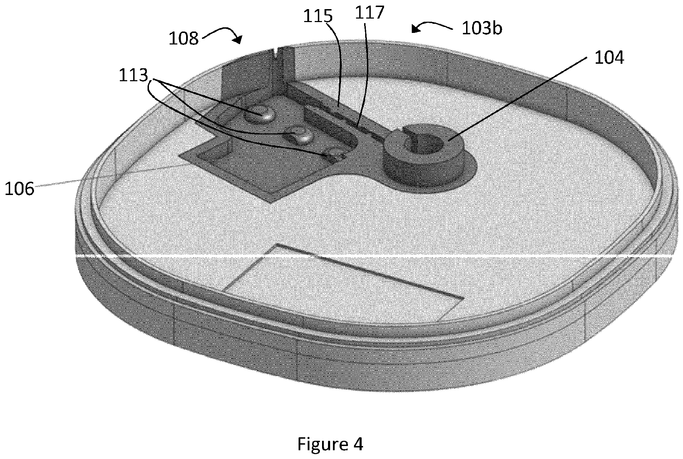

Figure 4 illustratively shows a view of a segment 103b of the housing 103 that corresponds to that shown in Figures 1a-2b except in this case the cover 106, the raised portions 112, the outline and shape of the recess 108, including the channel 115 to the additional opening, and the supporting structure 104 are integral and made of the same elastically deformable material, for example an elastomer. A particular advantage of this configuration is that the material of the supporting structure 104 is waterproof and provides a strong enough seal such that no additional O-ring seal is required. This arrangement also provides better waterproofing for the opening covered by the cover 106 as the snug fit of integral elements into the corresponding shaping in the housing segment 103b prevent the cover 106 from coming loose. The channel 115 in Figure 4 is also provided with one or more gripping teeth 117 to ensure the analyte sensor 101 is held securely in the recess 108.

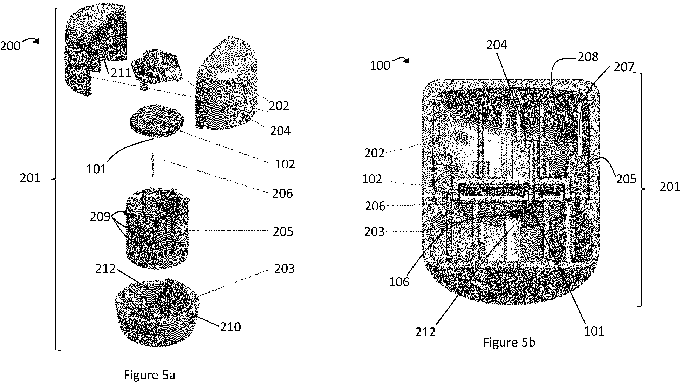

Figure 5a illustratively shows an exploded view of an analyte monitoring system 200 according to the present disclosure. The analyte monitoring system 200 comprises the analyte sensor 101 and sensor module 102 of any of Figures 1a-4 but additionally comprises an inserter device 201. The inserter device 201 comprises a body 202 and a removable cap 203 covering a forward end thereof (the term forward referring to the direction that faces the user's skin during use as opposed to rearward which refers to the opposite direction). The body 202 may comprise two or more injection moulded parts which are joined together during assembly.

Inside the space enclosed by the body 202 and removable cap 23, the inserter device 201 further comprises a carriage 204 for receiving the sensor module 102 therein before use, and a carriage release sleeve or cylinder 205 configured to release the carriage 204 upon movement of the cylinder 205 relative to the body 202.

The carriage 204 is provided with a cannula 206 or other corresponding sharp configured to hold and/or guide the analyte sensor 101 and pierce the skin as the carriage 204 with sensor module 102 is driven forwards to implant or insert the sensor 101 into the user's skin.

The internal surface of the body 202 may be provided with one or more guide rails 207 and/or hooks, recesses abutment surfaces 208 to guide the movement path of the cylinder 205 within the body by engagement with one or more corresponding slots, hooks, recesses, cam surfaces and/or abutment surfaces 209 on a surface of the cylinder 205.

For example, the cylinder 205 may be provided with one or more deformable wall sections with hooks that engage one or more corresponding a cam surface on the inner surface of the body 202. When a user presses the inserter device onto the skin or onto an adhesive patch on their skin after removing the cap 203, the cylinder 205 contacts the skin or patch and, as the user continues to press, the body 202 moves forwards relative to the cylinder 205 causing the one or more cam surfaces to push the hooks and deformable wall sections inwards until the end of the inward stroke is reached. At the end of the inward stroke, the hooks slide over the cam surfaces, releasing the deformable wall back into its original state and locking the carriage release sleeve in place inside the body 202, optionally providing a click or other sound indicating the end of the inward stroke and successful implantation of the sensor.

Alternatively, instead of a manual activation, the inserter device 103 may further comprise an optional force applicator (not shown) such as a spring or other force generating mechanism, coupled to the carriage 204. Pressing the cylinder 205 onto the patch 102 forces one or more hooks from one or more holding abutments inside the body 202 thereby releasing the carriage forward under the force of the force applicator and causing the analyte sensor to be implanted into the skin.

The inserter device 103 shown in Figures 5a and 5b is not provided with an automatic cannula retraction mechanism. Instead, the inserter device 103 is manually lifted up after use, thereby pulling the cannula 206 out of the skin but leaving the analyte sensor 101 implanted in the skin. However, it is also envisaged that an automatic cannula retraction mechanism may be provided, as will be appreciated by the skilled person.

The removable cap 203 is provided with one or more lugs 210 or hooks for engagement with a thread 211 in the body 202 of the inserter device. The engagement between the lugs 210 and the thread 211 converts a rotational movement of the cap during removal thereof from the body 202 into a slight rearward linear motion of the cap into the body. This causes one or more rearwardly extending arms 212 to move slightly rearwardly thereby applying a force to the surface of the exposed cover in the underside of the sensor module 102 to deform it, thereby starting up the controlling circuitry 107 of the sensor module 102.

Figure 5b illustratively shows a section view of the analyte monitoring system according to the present disclosure with an assembled inserter device 201 having the sensor module 102 and analyte sensor 101 received therein.

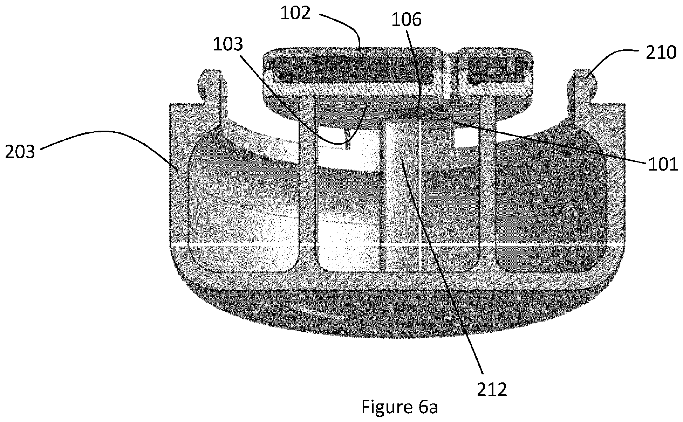

Figures 6a-6d illustratively show the operation of a switch-on mechanism of an analyte monitoring system, such as that illustrated in any of Figures 1a-5b, according to the present disclosure.

In Figure 6a, the removable cap 203 is 212 is positioned relative to the sensor module 101 so that the arm is blocked from moving rearwardly into the cover 106 by its contact with the housing 103 of the sensor module 103.

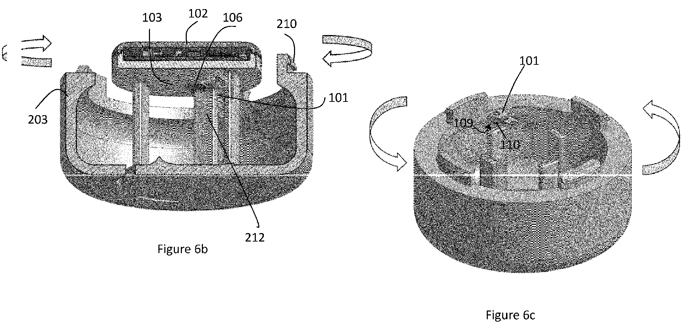

In Figure 6b, the removable cap 203 is rotated (indicated by the arrows), causing the lugs 210 to move along a path determined by the threading in the body (not shown) to convert some of the rotational movement into a rearward linear movement relative to the sensor module 102 and body. Because of the rotation, the arm is no longer blocked from pushing into the cover 106 and the rearward linear movement of the cap 203 pushes the arm 212 into the cover 106 to thereby deform it. This in turn pushes the deformable portion 109 of the analyte sensor 101 and the contact pad 110 thereon temporarily into contact with a corresponding contact pad on the underside of the PCB, to thereby activate a momentary switch of the circuitry and thereby switch on the sensor module.

Figure 6c corresponds to Figure 6b but from a different view, particularly showing the position of the analyte sensor 101 and deformable portion 109 with contact pad thereon.

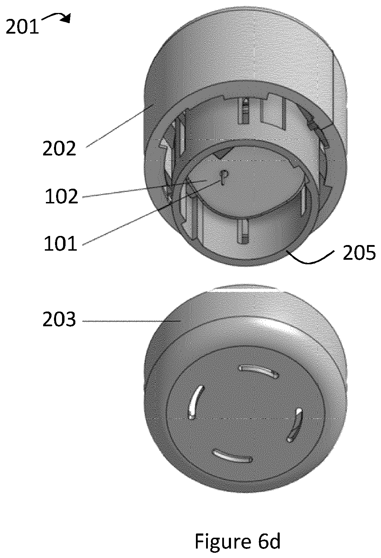

Figure 6d illustrates that, when the cap 203 is removed and the sensor module 102 switched on, the inserter device 201 is ready to be activated by pushing the cylinder 205 onto the user's skin, or onto an adhesive patch on the skin, and applying a force onto the body 202 to implant the analyte sensor 101 into the skin of a patient and to secure the now-switched-on sensor module onto the surface of the skin.

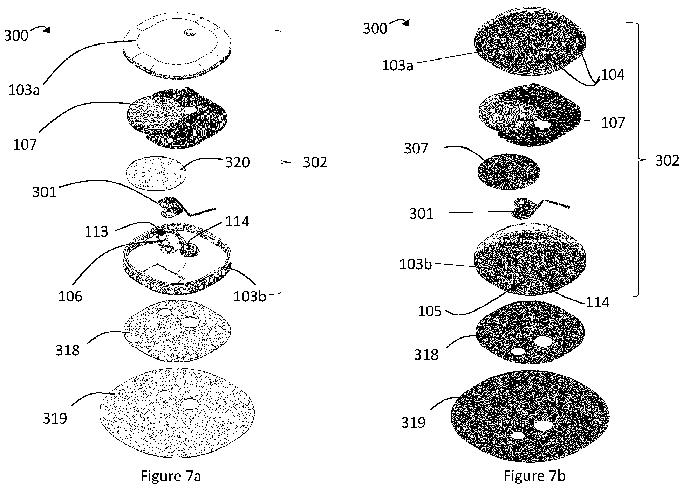

Figure 7a illustratively shows an exploded view of a further analyte monitoring system 300 according to the present disclosure. Many features of analyte monitoring system 300 correspond to those of analyte monitoring system 100, and like reference numerals are provided. The various discussions of features relating to analyte monitoring system 100 apply equally to the corresponding features of analyte monitoring system 300. In particular, the analyte monitoring system 300 comprises a skin implantable analyte sensor 301, and a sensor module 302 coupled to the skin-implantable analyte sensor 301. Also shown in Figure 7a is an optional battery adhesive 320 for securing the battery and/or circuitry 107 to the housing 103, along with an assembly adhesive 318 and a skin adhesive 319 for securing the sensor module 302 to a user.

Material 113 may be co-molded into a recess in the housing segment 103b, or otherwise inserted into the recess as described above.

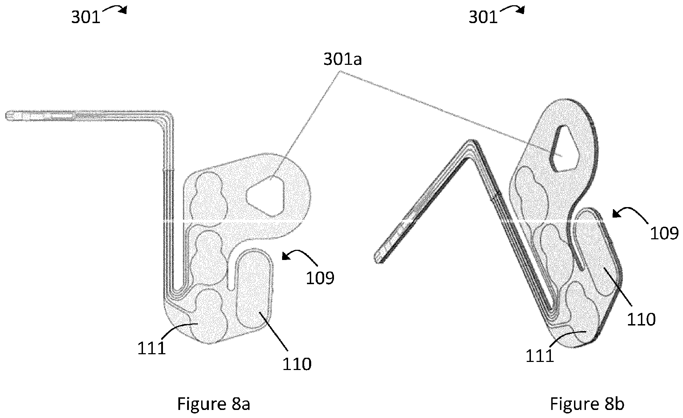

Figures 8a and 8b show views of the analyte sensor 301 for analyte monitoring system 300. As with analyte sensor 101, analyte sensor 301 comprises a pointed portion for insertion into a user's skin and a flat portion comprising deformable portion 109, contact pad 110 and additional contact pads 111. These features of analyte sensor 301 function as described in relation to analyte sensor 101.

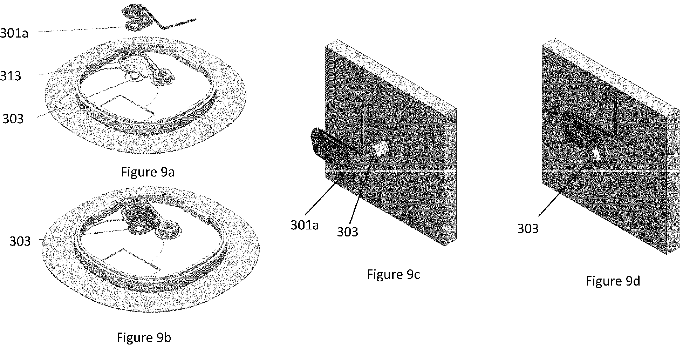

Relative to analyte sensor 101, analyte sensor 301 additionally comprises an aperture, opening or eyelet 301a. The eyelet 301a may assist with correctly positioning the analyte sensor during the manufacturing process, and further in securing the analyte sensor within the housing 103 both post-manufacture and during use of the sensor module. As shown in Figures 9a-d, the housing segment 103b may comprise a post or protrusion 303 configured for insertion into the eyelet 301a of the analyte sensor 301. The eyelet 301a and protrusion 303 may have corresponding shapes. While a triangular eyelet is depicted, it will be understood that any other shapes may also be provided for the eyelet and protrusion, including but not limited to a circle, oval, ellipse, triangle, rectangle, diamond, pentagon, etc. Advantageously, shapes with one or more corners (such as triangles) may be particularly effective at maintaining a suitable position for the analyte sensor 301 during and post-manufacturing. Optionally, the eyelet and protrusion may be provided with an irregular shape, such that the eyelet 301a can only receive the protrusion 303 when the analyte sensor 301 is in the desired orientation.

As further shown in Figures 9a and b, the material 113 may additionally comprise a recessed region 313 corresponding to the flat portion of the analyte sensor 301, to further secure the analyte sensor 301 within the housing 103.

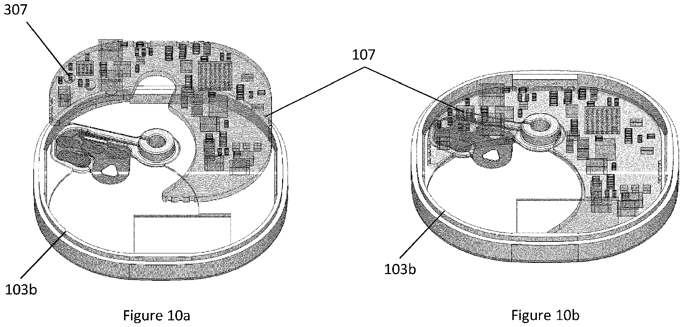

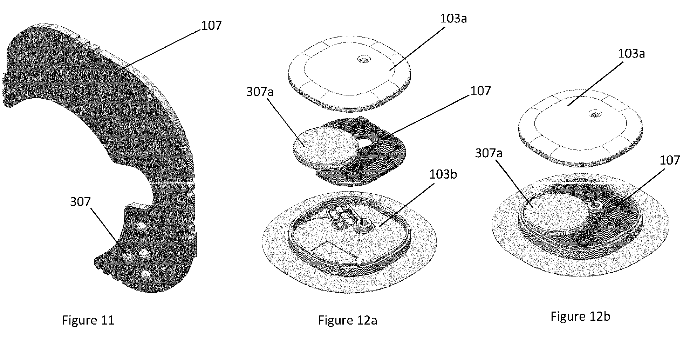

Figures 10a and b depict the insertion of the circuitry 107 into housing segment 103b after the analyte sensor is secured, e.g. by protrusion 303 and recess 313. As more clearly depicted in Figure 11, circuitry 107 may comprise more or more conductive dots 307 corresponding to the contact pad 110 and additional contact pads 111 of the analyte sensor.

Figures 12a and b show a further example of an insertion of circuitry 107 along with a corresponding battery 307a into housing segment 103b after the analyte sensor is secured. In a final step, housing segment 103a is secured to the housing segment 103b.

Other effective alternatives will occur to the skilled person. It will be understood that the invention is not limited to the described embodiments and encompasses modifications apparent to those skilled in the art lying within the scope of the claims appended hereto.

For example, the term contact pads as used herein may refer to any layer of conductive material including PCB traces and/or areas with solder material deposited thereon, for example with a solder mask.

For example, the shape of the analyte sensor described herein is generally said to have a shape having a pointed end for implanting into the user's skin and a flat portion having contact pads thereon, the flat portion being generally perpendicular or at an angle to the pointed portion. However, it will be appreciated that other shapes are also envisaged.

For example, whilst a generally square shaped opening is shown in the Figures, it is envisaged that other shapes may also be used including, for example, the above-described partial ring-like shape and others.

For example, while a spring-less inserter device is described above, an inserter device which uses a spring to apply the desired force to drive the sensor module and analyte sensor towards the skin may also be used.

For example, the elastically deformable cover may be manufactured separately from the sensor module and bonded to an inside surface therewith with an adhesive, alternatively, it may be manufactured as part of a two-shot injection moulding process at the same as the injection moulding of the sensor housing.

## Claims

1. An analyte monitoring system (100) comprising:
a skin-implantable analyte sensor (101); and
a sensor module (102) coupled to the skin-implantable analyte sensor,
wherein the sensor module comprises:
a housing (103) having an opening (105) in a surface thereof;
a cover (106) for covering the opening, the cover comprising an elastically deformable material; and
circuitry (107) disposed in the housing, and
wherein, responsive to application of a force on a surface of the cover, the cover is configured to elastically deform into the housing to activate the circuitry;
wherein the opening in the housing is in a surface of the housing that faces the skin of a user when in use.

2. The analyte monitoring system of claim 1, wherein after said application of said force ends, the elastically deformed cover is configured to return to its original shape, substantially flush with said surface of the housing.

3. The analyte monitoring system of any preceding claim, wherein the circuitry comprises a momentary switch activated by the elastic deformation of the elastically deformable cover into the housing, and wherein the circuitry is configured to perform a start-up routine responsive to activation of the momentary switch.

4. The analyte monitoring system of claim 3, wherein the analyte sensor comprises a deformable portion (109) configured to be deformed into contact with the circuitry responsive to a force applied thereto by the elastically deformable cover, thereby activating the momentary switch; and
optionally wherein the deformable portion of the analyte sensor comprises a contact pad (110), wherein the momentary switch comprises a contact pad, and wherein said momentary switch is configured to be activated by contact between the contact pad of the deformable portion of the analyte sensor and the contact pad of the momentary switch.

5. The analyte monitoring system of any preceding claim, wherein an inside surface of the housing comprises a recess (108) partially contiguous with an edge of the opening in the housing, the recess configured to receive a portion of the analyte sensor therein.

6. The analyte monitoring system of claim 5 when dependent on claim 4, the deformable portion of the analyte sensor when positioned in the recess is disposed over the opening in the housing.

7. The analyte monitoring system of claims 5 or 6, wherein a surface of the recess and/or an inside surface of the cover comprises one or more raised portions (112) configured to provide a reaction force against the analyte sensor when the analyte sensor is pressed into the recess and/or onto the inside surface of the cover.

8. The analyte monitoring system of claim 7, comprising a compressible layer of material (113) positioned over at least part of the recess and configured to contribute to the reaction force and to distribute the reaction force across a surface of the analyte sensor; and optionally wherein at least one of:
(i) the housing has a further opening configured for receiving a portion of the analyte sensor therethrough, and wherein the further opening is sealed with an O-ring seal (116); and
(ii) the compressible layer and/or, where present, the O-ring seal are integral with and comprise the same material as the cover.

9. The analyte monitoring system of any preceding claim, further comprising:
an inserter device (201) configured to implant the analyte sensor into the skin of a user and position the sensor module onto a surface of the skin of the user, the inserter device comprising a body (202) and a removable cap (203),
wherein a space enclosed by the body and the cap is configured to receive the sensor module and analyte sensor therein before use, and
wherein the removable cap comprises an arm (212) configured to apply a force to a surface of the cover of the sensor during removal of the cap from the body to elastically deform the cover into the housing to activate the circuitry.

10. The analyte monitoring system of claim 9, wherein at least one of:
(i) the removable cap is configured to rotate relative to the body of the inserter device during removal to cause the arm to apply said force to the surface of the cover, optionally wherein the body comprises a thread (211) on an inside surface thereof configured for engagement with a lug (210) on the removable cap, and wherein during removal of the removable cap by rotation, the lug is configured to follow the thread thereby moving the removable cap and arm in a direction towards the sensor module and applying a force with the arm to said surface of the cover to activate the circuitry; and
(ii) the inserter device further comprises a carriage (204) for receiving the sensor module therein before use, and a carriage release sleeve, the carriage release sleeve being configured to release the carriage under a force to move the sensor module onto the surface of the skin of the user.

11. The analyte monitoring system of any preceding claim, wherein the analyte sensor comprises an aperture (301a) configured to receive a protrusion (303) of the housing, to thereby positionally secure the analyte sensor within the housing; and
optionally wherein a shape of the aperture comprises one or more corners, and optionally wherein the aperture is triangular.

12. A sensor module (102) for use with an analyte monitoring system (100) with a skin-implantable analyte sensor (101), the sensor module comprising:
a housing (103) having an opening (105) in a surface thereof;
a cover (106) for covering the opening, the cover comprising an elastically deformable material; and
circuitry (107) disposed in the housing, and
wherein, responsive to application of a force on a surface of the cover, the cover is configured to elastically deform into the housing to activate the circuitry
wherein the opening in the housing is in a surface of the housing that faces the skin of a user when in use.

13. The sensor module of claim 12, wherein, after said application of said force ends, the elastically deformed cover is configured to return to its original shape, substantially flush with said surface of the housing.

14. A method of activating a sensor module (102) of an analyte monitoring system (100) with a skin-implantable analyte sensor (101) and inserter device (201), wherein the sensor module comprises:
a housing (103) having an opening (105) in a surface thereof, the opening in the housing being in a surface of the housing that faces the skin of a user when in use,
a cover (106) for covering the opening, the cover comprising an elastically deformable material; and
circuitry (107) disposed in the housing, and
wherein the method comprises applying a force to a surface of the cover to elastically deform the cover into the housing to activate the circuitry.

15. The method of claim 14, wherein the analyte monitoring system comprises an inserter device configured to implant the analyte sensor into the skin of a user and position the sensor module onto a surface of the skin of the user, wherein the inserter device comprises a body (202) and a removable cap (203) enclosing a space for receive the sensor module and analyte sensor therein before use, the method comprising:
applying said force to the surface of the cover during removal of the removable cap from the body by rotating the removable cap relative to the body to force an arm (212) of removable cap against the cover.

## Patentansprüche

1. Analytüberwachungssystem (100), umfassend:
einen in die Haut implantierbaren Analytsensor (101); und
ein Sensormodul (102), das mit dem in die Haut implantierbaren Analytsensor gekoppelt ist,
wobei das Sensormodul Folgendes umfasst:
ein Gehäuse (103) mit einer Öffnung (105) in einer seiner Oberflächen;
eine Abdeckung (106) zum Abdecken der Öffnung, wobei die Abdeckung ein elastisch verformbares Material umfasst; und
eine Schaltung (107), die im Gehäuse angeordnet ist, und
wobei die Abdeckung konfiguriert ist, sich bei Einwirkung einer Kraft auf eine Oberfläche der Abdeckung elastisch in das Gehäuse zu verformen, um die Schaltung zu aktivieren;
wobei sich die Öffnung im Gehäuse in einer Oberfläche des Gehäuses befindet, die bei Verwendung der Haut eines Benutzers zugewandt ist.

2. Analytüberwachungssystem nach Anspruch 1, wobei die elastisch verformte Abdeckung konfiguriert ist, nach Beenden der Krafteinwirkung in ihre ursprüngliche Form zurückzukehren, die im Wesentlichen bündig mit der Oberfläche des Gehäuses abschließt.

3. Analytüberwachungssystem nach einem vorstehenden Anspruch, wobei die Schaltung einen durch die elastische Verformung der elastisch verformbaren Abdeckung in das Gehäuse aktivierten Momentschalter umfasst und wobei die Schaltung so konfiguriert ist, dass sie bei Aktivierung des Momentschalters eine Startroutine durchführt.

4. Analytüberwachungssystem nach Anspruch 3, wobei der Analytsensor einen verformbaren Abschnitt (109) umfasst, der so konfiguriert ist, dass er durch eine von der elastisch verformbaren Abdeckung ausgeübte Kraft in Kontakt mit der Schaltung verformt wird und dadurch den Momentschalter aktiviert; und
wobei optional der verformbare Abschnitt des Analytsensors eine Kontaktfläche (110) umfasst, wobei der Momentschalter eine Kontaktfläche umfasst und wobei der Momentschalter so konfiguriert ist, dass er durch Kontakt zwischen der Kontaktfläche des verformbaren Abschnitts des Analytsensors und der Kontaktfläche des Momentschalters aktiviert wird.

5. Analytüberwachungssystem nach einem vorstehenden Anspruch, wobei eine Innenfläche des Gehäuses eine Aussparung (108) aufweist, die teilweise an eine Kante der Öffnung im Gehäuse angrenzt, wobei die Aussparung so konfiguriert ist, dass sie einen Abschnitt des Analytsensors darin aufnimmt.

6. Analytüberwachungssystem nach Anspruch 5, wenn von Anspruch 4 abhängig, wobei der verformbare Abschnitt des Analytsensors, wenn er in der Aussparung positioniert ist, über der Öffnung im Gehäuse angeordnet ist.

7. Analytüberwachungssystem nach Anspruch 5 oder 6, wobei eine Oberfläche der Aussparung und/oder eine Innenfläche der Abdeckung einen oder mehrere erhöhte Abschnitte (112) aufweist, die so konfiguriert sind, dass sie beim Eindrücken des Analytsensors in die Aussparung eine Reaktionskraft gegen diesen und/oder gegen die Innenfläche der Abdeckung bereitstellen.

8. Analytüberwachungssystem nach Anspruch 7, umfassend eine komprimierbare Materialschicht (113), die über mindestens einem Teil der Aussparung angeordnet ist und so konfiguriert ist, dass sie zur Reaktionskraft beiträgt und die Reaktionskraft über eine Oberfläche des Analytsensors verteilt; und wobei optional mindestens eines der folgenden Merkmale vorliegt:
(i) das Gehäuse weist eine weitere Öffnung auf, die zum Aufnehmen eines Abschnitts des Analytsensors konfiguriert ist, und wobei die weitere Öffnung mit einer O-Ringdichtung (116) abgedichtet ist; und
(ii) die komprimierbare Schicht und/oder, sofern vorhanden, die O-RingDichtung sind integraler Bestandteil der Abdeckung und umfassen das gleiche Material wie die Abdeckung.

9. Analytüberwachungssystem nach einem vorstehenden Anspruch, ferner umfassend:
eine Einführvorrichtung (201), die so konfiguriert ist, dass sie den Analytsensor in die Haut eines Benutzers implantiert und das Sensormodul auf einer Oberfläche der Haut des Benutzers positioniert, wobei die Einführvorrichtung einen Körper (202) und eine abnehmbare Kappe (203) umfasst,
wobei ein vom Körper und der Kappe umschlossener Raum so konfiguriert ist, dass er das Sensormodul und den Analytsensor vor Verwendung darin aufnimmt, und
wobei die abnehmbare Kappe einen Arm (212) umfasst, der so konfiguriert ist, dass er beim Abnehmen der Kappe vom Körper eine Kraft auf eine Oberfläche der Abdeckung des Sensors ausübt, um die Abdeckung elastisch in das Gehäuse zu verformen und so die Schaltung zu aktivieren.

10. Analytüberwachungssystem nach Anspruch 9, wobei mindestens eines der folgenden Merkmale vorliegt:
(i) die abnehmbare Kappe ist so konfiguriert, dass sie sich beim Abnehmen relativ zum Körper der Einführvorrichtung dreht, wodurch der Arm die Kraft auf die Oberfläche der Abdeckung ausübt, wobei der Körper optional ein Gewinde (211) an seiner Innenfläche umfasst, das zum Eingriff mit einem Ansatz (210) an der abnehmbaren Kappe konfiguriert ist, und wobei der Ansatz konfiguriert ist, beim Abnehmen der abnehmbaren Kappe durch Drehung dem Gewinde zu folgen und dadurch die abnehmbare Kappe und den Arm in Richtung des Sensormoduls zu bewegen und mit dem Arm eine Kraft auf die Oberfläche der Abdeckung auszuüben, um die Schaltung zu aktivieren; und
(ii) die Einführvorrichtung ferner einen Schlitten (204) zum Aufnehmen des Sensormoduls vor Verwendung sowie eine Schlittenfreigabehülse umfasst, wobei die Schlittenfreigabehülse so konfiguriert ist, dass sie den Schlitten unter Krafteinwirkung freigibt, um das Sensormodul auf die Hautoberfläche des Benutzers zu bewegen.

11. Analytüberwachungssystem nach einem vorstehenden Anspruch, wobei der Analytsensor eine Öffnung (301a) umfasst, die so konfiguriert ist, dass sie einen Vorsprung (303) des Gehäuses aufnimmt, um den Analytsensor innerhalb des Gehäuses in seiner Position zu sichern; und
wobei eine Form der Öffnung optional eine oder mehrere Ecken umfasst und wobei die Öffnung optional dreieckig ist.

12. Sensormodul (102) zur Verwendung mit einem Analytüberwachungssystem (100) mit einem in der Haut implantierbaren Analytsensor (101), wobei das Sensormodul Folgendes umfasst:
ein Gehäuse (103) mit einer Öffnung (105) in einer seiner Oberflächen;
eine Abdeckung (106) zum Abdecken der Öffnung, wobei die Abdeckung ein elastisch verformbares Material umfasst; und
eine Schaltung (107), die im Gehäuse angeordnet ist, und
wobei die Abdeckung konfiguriert ist, sich bei Einwirkung einer Kraft auf eine Oberfläche der Abdeckung elastisch in das Gehäuse zu verformen, um die Schaltung zu aktivieren
wobei sich die Öffnung im Gehäuse in einer Oberfläche des Gehäuses befindet, die bei Verwendung der Haut eines Benutzers zugewandt ist.

13. Sensormodul nach Anspruch 12, wobei die elastisch verformte Abdeckung konfiguriert ist, nach Beenden der Krafteinwirkung in ihre ursprüngliche Form zurückzukehren, die im Wesentlichen bündig mit der Oberfläche des Gehäuses abschließt.

14. Verfahren zur Aktivierung eines Sensormoduls (102) eines Analytüberwachungssystems (100) mit einem in die Haut implantierbaren Analytsensor (101) und einer Einführvorrichtung (201), wobei das Sensormodul Folgendes umfasst:
ein Gehäuse (103) mit einer Öffnung (105) in einer seiner Oberflächen, wobei sich die Öffnung im Gehäuse in einer Oberfläche des Gehäuses befindet, die bei Verwendung der Haut eines Benutzers zugewandt ist,
eine Abdeckung (106) zum Abdecken der Öffnung, wobei die Abdeckung ein elastisch verformbares Material umfasst; und
eine Schaltung (107), die im Gehäuse angeordnet ist, und
wobei das Verfahren Ausüben einer Kraft auf eine Oberfläche der Abdeckung umfasst, um die Abdeckung elastisch in das Gehäuse zu verformen, um die Schaltung zu aktivieren.

15. Verfahren nach Anspruch 14, wobei das Analytüberwachungssystem eine Einführvorrichtung umfasst, die so konfiguriert ist, dass sie den Analytsensor in die Haut eines Benutzers implantiert und das Sensormodul auf einer Oberfläche der Haut des Benutzers positioniert, wobei die Einführvorrichtung einen Körper (202) und eine abnehmbare Kappe (203) umfasst, die einen Raum zum Aufnehmen des Sensormoduls und des Analytsensors darin vor Verwendung umschließt, wobei das Verfahren Folgendes umfasst:
Ausüben der Kraft auf die Oberfläche der Abdeckung, während die abnehmbare Kappe vom Körper abgenommen wird, durch Drehen der abnehmbaren Kappe relativ zum Körper, um einen Arm (212) der abnehmbaren Kappe gegen die Abdeckung zu drücken.

## Revendications

1. Système de surveillance d'analyte (100) comprenant :
un capteur d'analyte implantable dans la peau (101) ; et
un module capteur (102) couplé au capteur d'analyte implantable dans la peau,
dans lequel le module capteur comprend :
un boîtier (103) présentant une ouverture (105) dans une surface de celui-ci ;
un couvercle (106) pour recouvrir l'ouverture, le couvercle étant constitué d'un matériau élastiquement déformable ; et
une circuiterie (107) disposée dans le boîtier, et
dans lequel, en réponse à l'application d'une force sur une surface du couvercle, le couvercle est configuré pour se déformer élastiquement dans le boîtier afin d'activer la circuiterie ;
dans lequel l'ouverture du boîtier se trouve dans une surface de celui-ci qui est en contact avec la peau d'un utilisateur lors de son utilisation.

2. Système de surveillance d'analyte selon la revendication 1, dans lequel, après la fin de ladite application de ladite force, le couvercle déformé élastiquement est configuré pour reprendre sa forme initiale, sensiblement au même niveau que ladite surface du boîtier.

3. Système de surveillance d'analyte selon une quelconque revendication précédente, dans lequel la circuiterie comprend un commutateur momentané activé par la déformation élastique du couvercle élastiquement déformable dans le boîtier, et dans lequel la circuiterie est configurée pour réaliser une routine de démarrage en réponse à l'activation du commutateur momentané.

4. Système de surveillance d'analyte selon la revendication 3, dans lequel le capteur d'analyte comprend une partie déformable (109) configurée pour se déformer en contact avec la circuiterie en réponse à une force qui lui est appliquée par le couvercle élastiquement déformable, activant ainsi le commutateur momentané ; et
facultativement dans lequel la partie déformable du capteur d'analyte comprend une pastille de contact (110), dans lequel le commutateur momentané comprend une pastille de contact, et dans lequel ledit commutateur momentané est configuré pour être activé par contact entre la pastille de contact de la partie déformable du capteur d'analyte et la pastille de contact du commutateur momentané.

5. Système de surveillance d'analyte selon une quelconque revendication précédente, dans lequel une surface intérieure du boîtier comprend un évidement (108) partiellement contigu à un bord de l'ouverture dans le boîtier, l'évidement étant configuré pour recevoir une partie du capteur d'analyte.

6. Système de surveillance d'analyte selon la revendication 5, lorsqu'elle dépend de la revendication 4, dans lequel la partie déformable du capteur d'analyte, lorsqu'il est positionné dans l'évidement, est disposée au-dessus de l'ouverture du boîtier.

7. Système de surveillance d'analyte selon la revendication 5 ou 6, dans lequel une surface de l'évidement et/ou une surface intérieure du couvercle comprennent une ou plusieurs parties surélevées (112) configurées pour exercer une force de réaction contre le capteur d'analyte lorsque celui-ci est inséré dans l'évidement et/ou sur la surface intérieure du couvercle.

8. Système de surveillance d'analyte selon la revendication 7, comprenant une couche compressible de matériau (113) positionnée au-dessus d'au moins une partie de l'évidement et configurée pour contribuer à la force de réaction et la répartir sur une surface du capteur d'analyte ; et, facultativement, dans lequel au moins l'un parmi :
(i) le boîtier présente une ouverture supplémentaire configurée pour recevoir une partie du capteur d'analyte à travers celle-ci, et dans lequel l'ouverture supplémentaire est scellée par un joint torique (116) ; et
(ii) la couche compressible et/ou, lorsqu'il est présent, le joint torique font partie intégrante du couvercle et sont constitués du même matériau que celui-ci.

9. Système de surveillance d'analyte selon une quelconque revendication précédente, comprenant en outre :
un dispositif d'insertion (201) configuré pour implanter le capteur d'analyte dans la peau d'un utilisateur et positionner le module capteur sur une surface de la peau de l'utilisateur, le dispositif d'insertion comprenant un corps (202) et un capuchon amovible (203),
dans lequel un espace délimité par le corps et le capuchon est configuré pour recevoir le module capteur et le capteur d'analyte dans celui-ci avant utilisation, et
dans lequel le capuchon amovible comprend un bras (212) configuré pour appliquer une force à une surface du couvercle du capteur lors du retrait du capuchon du corps afin de déformer élastiquement le couvercle dans le boîtier pour activer la circuiterie.

10. Système de surveillance d'analyte selon la revendication 9, dans lequel au moins l'un parmi :
(i) le capuchon amovible est configuré pour tourner par rapport au corps du dispositif d'insertion lors de son retrait pour amener le bras à appliquer ladite force à la surface du couvercle, facultativement dans lequel le corps comprend un filetage (211) sur une surface intérieure de celui-ci configuré pour s'engager avec une patte (210) sur le capuchon amovible, et dans lequel, lors du retrait du capuchon amovible par rotation, la patte est configurée pour suivre le filetage, déplaçant ainsi le capuchon amovible et le bras en direction du module capteur et appliquant une force avec le bras à ladite surface du couvercle pour activer la circuiterie ; et
(ii) le dispositif d'insertion comprend en outre un chariot (204) destiné à recevoir le module capteur dans celui-ci avant utilisation, et un manchon de libération de chariot, le manchon de libération de chariot étant configuré pour libérer le chariot sous l'effet d'une force pour déplacer le module capteur sur la surface de la peau de l'utilisateur.

11. Système de surveillance d'analyte selon une quelconque revendication précédente, dans lequel le capteur d'analyte comprend un orifice (301a) configuré pour recevoir une protubérance (303) du boîtier, afin de fixer le capteur d'analyte en place à l'intérieur du boîtier ; et
facultativement dans lequel la forme de l'orifice comprend un ou plusieurs coins, et facultativement dans lequel l'orifice est triangulaire.

12. Module capteur (102) destiné à être utilisé avec un système de surveillance d'analyte (100) présentant un capteur d'analyte implantable dans la peau (101), le module capteur comprenant :
un boîtier (103) présentant une ouverture (105) dans une surface de celui-ci ;
un couvercle (106) pour recouvrir l'ouverture, le couvercle étant constitué d'un matériau élastiquement déformable ; et
une circuiterie (107) disposée dans le boîtier, et
dans lequel, en réponse à l'application d'une force sur une surface du couvercle, le couvercle est configuré pour se déformer élastiquement dans le boîtier afin d'activer la circuiterie,
dans lequel l'ouverture du boîtier se trouve dans une surface de celui-ci qui est en contact avec la peau d'un utilisateur lors de son utilisation.

13. Module capteur selon la revendication 12, dans lequel, après la fin de ladite application de ladite force, le couvercle déformé élastiquement est configuré pour reprendre sa forme initiale, sensiblement au même niveau que ladite surface du boîtier.

14. Procédé d'activation d'un module capteur (102) d'un système de surveillance d'analyte (100) avec un capteur d'analyte implantable dans la peau (101) et un dispositif d'insertion (201), dans lequel le module capteur comprend :
un boîtier (103) présentant une ouverture (105) dans une surface de celui-ci, l'ouverture du boîtier étant située dans une surface du boîtier qui fait face à la peau de l'utilisateur lors de son utilisation,
un couvercle (106) pour recouvrir l'ouverture, le couvercle étant constitué d'un matériau élastiquement déformable ; et
une circuiterie (107) disposée dans le boîtier, et
dans lequel le procédé comprend l'application d'une force à une surface du couvercle pour déformer élastiquement le couvercle dans le boîtier afin d'activer la circuiterie.

15. Procédé selon la revendication 14, dans lequel le système de surveillance d'analyte comprend un dispositif d'insertion configuré pour implanter le capteur d'analyte dans la peau d'un utilisateur et positionner le module capteur sur une surface de la peau de l'utilisateur, dans lequel le dispositif d'insertion comprend un corps (202) et un capuchon amovible (203) renfermant un espace destiné à recevoir le module capteur et le capteur d'analyte dans celui-ci avant utilisation, le procédé comprenant :
l'application de ladite force à la surface du couvercle lors du retrait du capuchon amovible du corps en faisant tourner le capuchon amovible par rapport au corps pour forcer un bras (212) du capuchon amovible contre le couvercle.
